# EUROPEAN PATENT SPECIFICATION

(11) **EP 1 696 888 B1**
(45) Date of publication and mention of the grant of the patent: **08.04.2015**
(21) Application number: 04806077.6
(22) Date of filing: 15.12.2004
(51) Int. Cl.: A61K 9/16, A61K 31/573, A61K 31/4164, A61K 38/22

(54) **PROCESS FOR PRODUCING PELLETS FOR PHARMACEUTICAL COMPOSITIONS**
VERFAHREN ZUR HERSTELLUNG VON PELLETS FÜR PHARMAZEUTISCHE ZUSAMMENSETZUNGEN
PROCEDE PERMETTANT DE FORMER DES GRANULES POUR COMPOSITIONS PHARMACEUTIQUES

(30) Priority: 23.12.2003 GB 0329851; 23.12.2003 GB 0329854
(43) Date of publication of application: 06.09.2006
(73) Proprietor: TEMREL LIMITED, Road Town, Tortola (VG)
(72) Inventor: SPEIRS, Christopher, Surrey KT22 9NG (GB); MOIR, Peter, Country Waterford (IE); WILLIAMS, Richard, CH- 4052 Basel (CH); CLARK, Michael, Gloustershire GL50 2EA (GB)
(74) Representative: Beck Greener
(86) International application number: PCT/GB2004/005263
(87) International publication number: WO 2005/060938

(56) References cited:
- US-A- 5 834 021
- US-A1- 2001 004 458
- US-A1- 2003 018 000

## Description

The present invention relates to a process to produce particles, particularly for use in pharmaceutical compositions. In particular, the invention relates to the use of water to control particle size.

US-A-5834021 (Speirs; published on 10th November 1998) discloses a non-disintegratable solid enteric composition comprising 5 wt % prednisolone metasulphobenzoate ("Pred-MSB") in an excipient matrix comprising 40 wt % microcrystalline cellulose, 35 wt % lactose and 20 wt % croscarmellose sodium. The composition is in the form of pellets having a diameter in the range of 1000 to 1400 µm. The pellets are formed by dry mixing the Pred-MSB with the cellulose, the lactose and the croscarmellose sodium. Water is added to the mixture which is then stirred for 10 minutes to form an extrudable paste. The paste is extruded from a 25 mm diameter bowl through a 1 mm diameter tube of about 5 mm length at a rate of about 100 mm/min and spheronised on an 8 in (20 cm) plate rotated at about 1000 rpm for 10 to 15 minutes to provide said pellets. The resultant pellets are dried at 50°C for 30 min on a fluidised bed. The pellets are then coated with an Eudragit^{™} S100 (available from Röhm Pharma GmbH, Darmstadt, Germany) coating to provide a theoretical weight gain on coating of 11.6% and filled (15.7 mg per capsule) into size 1 hard gelatin capsules. The filled capsules are coated with an Eudragit^{™} L100 (also available from Röhm Pharma GmbH) coating to provide a theoretical weight gain on coating of 10.2%. The coated capsules may be used as a delayed and sustained release oral treatment of inflammatory bowel disease ("IBD").

Similar treatments of IBD are described in UK patent application Nos. 0215656.0 and 0215657.8 (Speirs; unpublished).

The diameter of the pellets is usually in the range between from about 500 to 2500 µm, preferably 800 to 1700 µm, more preferably 800 to 1500 µm and still more preferably 1000 to 1500 µm. However, it should be appreciated that pellets may have a diameter anywhere within the aforementioned ranges and that a capsule may have pellets having a range of diameters. One reason pellets of this size are preferred is that they may be coated satisfactorily with, for example, an enteric coating. Such enterically-coated pellets display the required release profile in the intestines. Smaller pellets tend to be less spherical and more elongated and may be below the required size to allow homogeneous filling of capsules while retaining a sufficient number to distribute through the bowel. The preferred size ranges have been justified by bioscintigraphy, the results of which showing that 200 or so pellets obtained an appropriate spread throughout the bowel.

The process disclosed in US-A-5834021 produces a range of pellet sizes. The pellets have to be screened so that the pellets of required size can be collected. Pellets that are either too large or too small to be used effectively in the delayed and sustained release capsules would normally be discarded resulting in significant wastage. Such wastage is obviously undesirable. There is a need therefore for an improved process that produces particles having a more favourable distribution of particle sizes that is more particles within the required diameter range, resulting in a reduction in the amount of wastage.

The inventors have discovered that even small variations, e.g. ±5 wt %, in the amount of water used in the above-mentioned process causes a significant change in the size of the particles and the distribution of particle sizes. With this in mind, the inventors reasoned that particle size and, more importantly, particle size distribution is dependant on the amount of water used. The inventors realised that the amount of water could, therefore, be used to control the particle size and distribution. In this way, particles having different ranges of sizes could be produced.

According to a first aspect of the present invention, there is provided a process for the production of particles for use in a pharmaceutical composition, said process comprising:
mixing water with a component composition comprising at least a rheology modifying agent to produce a paste;
extruding at least a portion of the paste to form extrudate;
spheronising at least a portion of the extrudate to form spheronised particles; and
drying at least a portion of the spheronised particles, characterised in that the process comprises:
   controlling the amount of water used with the component composition so as to obtain a particle size distribution such that about 80% to 90% of the particles have a diameter from about 800 to about 1500 µm.

The term "paste" is intended to include wet granulate.

The particles of the present invention are typically pellets or granules. In preferred embodiments, the composition further comprises sugar and cellulose.

Without wishing to be bound by any particular theory, the amount of water affects particle size due to the state of hydration of the matrix of the particle. Once the amount of water passes a certain point, the matrix is too wet and forms large agglomerates. It would appear that a large amount of water is taken up by the rheology modifying agent. Beyond the saturation point for this process, the amount of water appears critical.

One advantage of the present invention is that more particles having a diameter within the required range, usually 800 to 1500 µm, are produced. Different pluralities of preferred pellets of this size may be treated/coated using different modalities or thicknesses of delayed release coating material in order to achieve release at specified areas of the bowel. An example of such coated pluralities of pellets is disclosed in PCT/GB03/02911.

Such coated pluralities of pellets allow a number of clinical objectives to be met. For example, they allow continuous delivery of a drug to treat large areas of bowel where the drug would otherwise be absorbed or metabolised if suddenly released. In addition, they allow continuous delivery of a drug over a section of the bowel to increase contact with the absorptive mucosa thereby allowing maximum absorption whereas the drug would be broken down if otherwise released in one section. Further, where a drug at high concentration would be toxic to the gut mucosa, the pellets allow the drug to be continually available at low concentration thereby allowing absorption without or with reduced toxicity.

Water is usually used in an amount of between from about 180 wt % to about 190 wt % of the component composition and is preferably used in an amount of about 185 wt % of the component composition. The inventors found the amount of water used in the process to form the matrix by absorption to be surprisingly large. This large amount of water distinguishes the present invention over all other pelletting processes of which the inventors are aware.

From about 80 % to about 98 % of particles and, typically between from about 90 % to 98 % of particles, have a diameter between the range of about 800 to about 1500 microns. Even though the number of particles whose diameter is within the required range is greater than for the process disclosed in US-A-5834021, the dry particles may be screened to obtain particles having a diameter with the range of about 800 to about 1500 µm and to remove particles whose diameter does not fall within that range.

Use of about 5 wt % less water usually reduces particle size significantly. Conversely, use of 5 wt % more water increases particle size such that 100% of particles have a diameter greater than 1500 µm which is useless if the pellets are to be enterically coated and used to release an active into the bowels.

The use of less water reduces the particle size distribution such that fewer particles have a diameter within the desired 800 to 1500 µm range and the mean particle size is reduced. The use of more water increases particle size distribution until all pellets are greater than 1500 µm. Thus, preferred embodiments of the present invention increase the number of useful particles and reduces the amount of waste.

Pellets produced according to the invention are particularly applicable to the delivery of high molecular weight compounds, for example proteins or peptides, in which the integrity of the tertiary structure is critical to the efficacy and safety of the compound. A particular advantage of these pellets is that an oral pharmaceutical composition may be prepared under gentle conditions relative to most pharmaceutical processes, whilst providing a desired release profile of the compound in the intestinal tract.

An example of a high molecular weight compound, which would benefit from formulation in a composition of the present invention is erythropoietin, a glycosylated protein hormone and haematopoietic growth factor, which is considered useful in the management of anaemia in chronic renal failure among other conditions and has been investigated in the treatment of anaemia of inflammatory bowel disease as well as other normocytic-normochromic anaemias. Erythropoietin is conventionally administered subcutaneously or intravenously, although a tabletted form of erythropoietin has been disclosed (RU-A-2152206).

Other classes of high molecular weight compound which may benefit from the present invention include interferons, TNF antagonists and specific protein and polypeptide agonists and antagonists of the immune system, hormones, such as human growth hormone and cytokines and cytokine antagonists. Other high molecular weight compounds that might be used include vaccines.

Particles produced according to the invention are also particularly useful in the delivery of anti-infective compounds such as metronidazole. Such pellets achieve high concentrations of the anti-infective compounds in the lumen of the gut and at the gut wall and allow the anti-infective agent to be disseminated through an appropriate extended area of the gut. In addition, pellets comprising an anti-inflammatory agent also achieve a high concentration of the agent in the gut wall.

Other compounds and classes of compound whose administration may benefit from the present invention include analgesics and antipyretics; antibacterial and antiprotozoal agents, such as metronidazole, albenazole, mebendazole, prazinquantel and other nitroimidazole antibiotics and antibiotics active against anaerobic bacteria; clarithromycin and other macrolide antibiotics; gentamycin, ciprofloxacin, rifabutin and other such antibiotics active against infective organisms commonly associated with or causing disorders of the intestine; antifungal agents; antiinflammatory agents such as, salicylates, for example 5-aminosalicylic acid, 4-aminosalicylic acid and derivatives, such as balsalazide, steroids, especially prednisolone metasulphobenzoate; probiotics and prebiotics which have been shown to influence the symptoms of inflammatory bowel disease and irritable bowel syndrome and recovery from antibiotic-associated diarrhea. Similarly, pharmacologically active drug substances known to influence the symptoms of irritable bowel syndrome, particularly by affecting neurotransmission in the gut at local sites such as those affecting the serotinergic system and those active at the site of opiate receptors. α-amylase and paracetamol may also be administered using the composition of the present invention.

Other compounds which may benefit from the present invention include certain compounds that have toxic effects which limit their clinical usefulness, especially by causing local toxicity in specific areas of the gastrointestinal tract. Included among such compounds are examples of antibiotics, bisphosphonates and antiinflammatory drugs. A particular example is metformin, which is intolerable to many patients due to adverse effects on the gastrointestinal tract. The present invention may be utilised to minimise the concentration of the compound at the specific sites of toxicity and so allowing an effective therapeutic dose to be administered with a reduction in adverse events.

Antibiotics effective in the treatment of inflammatory bowel disease or infective disorders of the intestine are frequently toxic when absorbed and the present invention may be applied to administer them to their sites of action in the intestine, achieving sufficient local concentrations whilst minimising systemic uptake. Of particular application to the present invention are toxic antibiotics, such as gentamycin, particularly in patients predisposed to the toxic effects of such drugs such as those with renal dysfunction. Patients with chronic disorders of the intestine, for example Crohn's disease and pouchitis, requiring continued administration of certain antibiotics, for example, metronidazole, over long periods are likely to benefit particularly from the present invention.

Other possible actives include anticancer or cytotoxic agents such as cyclophosphamide, cisplatin and other platinum drugs and vincristine and other vinca alkaloids; immunomodulators such as methotrexate, azathioprine and cyclosporin; and anti-parasitic agents such as albenazole.

Pharmacologically acceptable salts and derivatives of the active compounds may also be used.

The preferred compounds for use in the present invention are prednisolone sodium metasulphobenzoate, 5-aminosalicylic acid, metronidazole, clarithromycin, metformin, paracetamol, α-amylase and erythropoietin. In the case of prednisolone pellets, the particles may be used to treat inflammatory bowel disease, for example, in a delayed and sustained release oral medicament.

The therapeutically active compound is preferably present in a therapeutically effective amount, usually between from more than 0 wt % to about 90 wt%, preferably between from more than 0 wt % to 40 wt %, of the component composition. The final amount of the active depends on the potency of the active. Therefore, actives that have relatively higher potency, for example erythropoietin, may be present in an amount between from more than 0 wt % to about 1 wt %. In addition, actives that have relatively lower potency, for example prednisolone or metronidazole, may be present in an amount between from about 5 wt % to about 20 wt %.

One preferred composition consists essentially of prednisolone or a pharmacologically acceptable salt (e.g. predisolone sodium metasulphobenzoate) or derivative thereof, rheology modifying agent, sugar and cellulose.

A second preferred composition consists essentially of metronidazole or a pharmacologically acceptable salt or derivative thereof, rheology modifying agent, sugar and cellulose.

A third preferred composition consists essentially of erythropoietin or a pharmacologically acceptable salt or derivative thereof, theology modifying agent, sugar and cellulose.

The rheology modifying agent is swells upon hydration to form a gel-like matrix having visco-elastic properties. When the pellets are dried, they do not shrink significantly. Therefore, the Inventors reason that, once the water is removed, a particular structure is formed which might be responsible for the release characteristics of the pellets. The rheology modifying agent is usually a hydrophilic gelling agent such as starch or hydropropyl-methylcellulose.

The rheology modifying agent may be, e.g. crospovidone, sodium starch glycolate or croscarmellose sodium, i.e. Ac-Di-Sol^{™} (FMC Biopolymer, 1735 Market Street, Philadelphia, PA 19103, USA). Croscarmellose sodium is usually used as a super disintegrant, i.e. a compound that assists dissolution of a composition. It is, therefore, surprising and totally unexpected that a super disintegrant would form a gel-like matrix. The rheology modifying agent is present in an amount of at least 5 wt % of the component composition, preferably at least 10 wt % and more preferably in an amount of between from about 10 to about 40 wt %, e.g. 20 wt %, of the component composition.

The sugar is preferably lactose monohydrate. The sugar is preferably present in an amount of between from about 30 to about 50 wt %, e.g. 35 wt %, of the component composition.

The cellulose is preferably microcrystalline cellulose. The cellulose is preferably present in an amount of between from about 35 to about 45 wt %, e.g. 30 wt %, of the component composition.

The speed of the spheroniser is very slow in comparison to that in known pellet manufacturing processes. For the purposes of the present invention, the spheronising plate usually rotates at between from about 125 rpm to 1800 rpm, preferably 200 rpm to 1000 rpm and, if the speed of rotation used is outside this range then the spheroniser usually fails to make pellets. In addition, with knowledge of known processes, the use of a smaller spheronising plate would intuitively require a faster rotation speed. However, in the present invention, the reverse is true and a smaller plate requires a faster speed of rotation. To the inventors' knowledge, this observation is unique in pellet manufacturing.

Controlling the amount of water used allows optimisation of the size distribution of particles at maximum process yields. The particles are intended for a particular purpose, for example medical treatment of a condition, e.g. IBD.

The resultant particles may be coated with an enteric coating such as Eudragit^{™} S which is an anionic copolymer of methacrylic acid and methacrylic acid methyl ester in which the ratio of free carboxylic groups to ester groups is approximately 1 : 2 and has a mean molecular weight of 135,000. A plurality of the coated particles may be encapsulated in a capsule or compressed into a tablet. The capsule or tablet may be coated with another enteric coating such as Eudragit^{™} L which differs from Eudragit S in that the ratio of free carboxylic groups to ester groups is approximately 1 : 1. Both Eudragit^{™} L and Eudragit^{™} S are insoluble in gastric juice (about pH 6) but only Eudragit^{™} L is readily soluble in intestinal juice below about pH 7. In this way, release of the active component is delayed until the colon and sustained to increase the effectiveness of the active. Sustained release is believed to be achieved at least in part through the coating becoming permeable.

It is believed currently that the gel-like matrix is formed from the cellulosic components of the pellets upon rehydration. In preferred embodiments, the cellulosic components are microcrystalline cellulose and croscarmellose sodium (a cellulose derivative). On rehydration, the pellets swell and release the active component in a sustained manner over time. The pellets also become "sticky" on rehydration and stick to the gut wall. As a result, the swollen pellets stick to the target site in the gut thereby increasing the effectiveness of the active. In addition, the pH within the gut increases from the centre of the gut lumen to the wall of the gut. Where the pellets are coated with a pH dependent release coating material, the rate of release of the active increases as the pellets approach the gut wall. This feature of preferred embodiments of the invention may also increase the effectiveness of the active.

The results also indicate that the overall yield (after drying) of the particles increases as the amount of water used approaches the optimum amount.

In a second aspect of the present invention, there is provided a process for the production of particles for use in a pharmaceutical composition, said process comprising the steps of:
mixing water with a component composition comprising at least a rheology modifying agent to produce a paste;
extruding at least a portion of the paste to form extrudate;
spheronising at least a portion of the extrudate to form spheronised particles; and
drying at least a portion of the spheronised particles,
wherein the amount of water used is between from about 180 to about 190 wt % of the weight of the component composition and, where the spheronising step uses a rotation 70 cm plate, the plate does not rotate at about 33 rpm.

The process of the second aspect may have any or all of the preferred features of the process defined above, in any appropriate combination.

Preferred embodiments of the present invention will now be described, by way of example only and with reference to the accompanying figures. In the figures:
Figure 1 is a photograph of uncoated pellets produced in Example 1;
Figure 2 is a photograph of uncoated pellets produced in Example 2; and
Figure 3 is a photograph of uncoated pellets produced in Example 3.

### Example 1 - 5 wt% prednisolone sodium metasulphobenzoate

Prednisolone metasulphobenzoate pellets were prepared by preparing a dry mix of 5 wt% prednisolone sodium metasulphobenzoate, 40 wt% microcrystalline cellulose (Avicel^{™} PH 101), 35 wt% lactose monohydrate (D80 200 Mesh) and 20 wt% croscarmellose sodium (Ac-Di-Sol^{™}). Purified water (185 wt% of the dry mix components) was added and the resulting mixture mixed for 10 minutes to form and extrudable paste which was then extruded and spheronised. The pellets were then dried in a fluid bed granulator and screened to ensure the size of the particles was in the range 800 to 1500 µm.

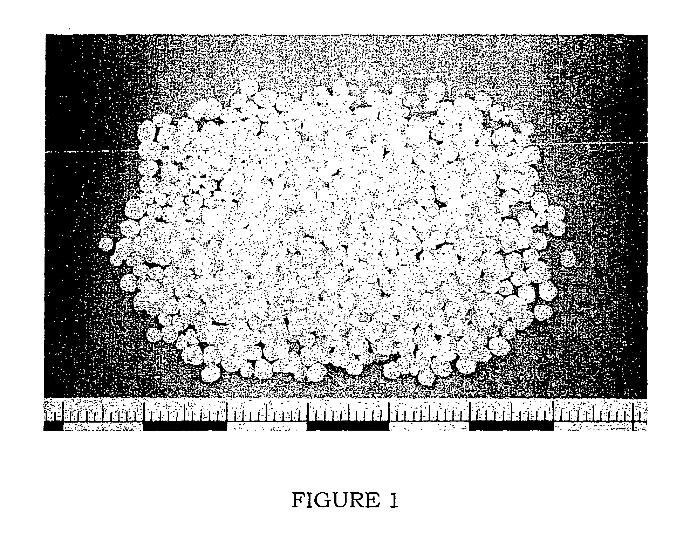

Figure 1 depicts the pellets formed by Example 1. The majority of these pellets are within the required range of 800 to 1500 µm.

### Example 2 - 5 wt% prednisolone sodium metasulphobenzoate

Pellets were formed using the steps described in Example 1 although only 180 wt % water was used instead of 185 wt %. The yield (after drying) of the pellets was 91 %.

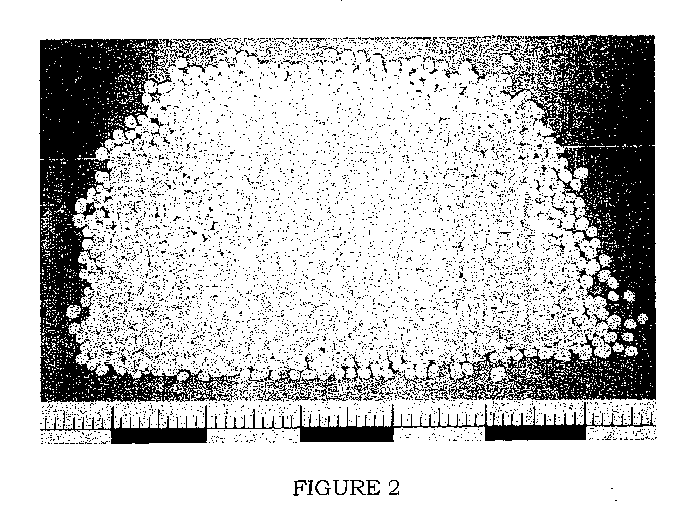

Figure 2 depicts the pellets formed by Example 2. The photograph clearly shows that the size of the pellets is reduced significantly when less water is used.

### Example 3 - 5 wt% prednisolone sodium metasulphobenzoate

Pellets were formed using the steps described in Example 1 although 190 wt % water was used instead of 185 wt %.

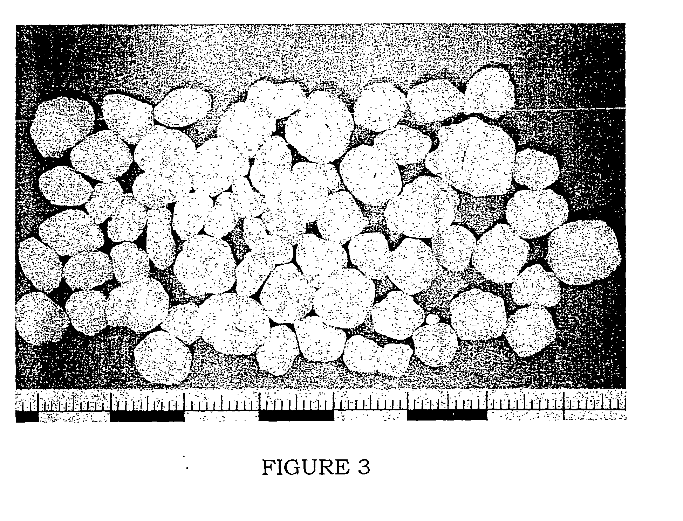

Figure 3 depicts the pellets formed by Example 3. The photograph clearly shows that the size of the pellets is increased significantly when more water is used.

### Example 4 - 5 wt% prednisolone sodium metasulphobenzoate

Pellets were formed using the steps described in Example 1 although only 182.5 wt % water was used instead of 185 wt %. The yield (after drying) of the pellets was 96.5 %.

### Example 5 - 5 wt% prednisolone sodium metasulphobenzoate

Pellets were formed using the steps described in Example 1 although only 177.5 wt % water was used instead of 185 wt %. The yield (after drying) of the pellets was 85 %.

### Example 6 - 20 wt% metronidazole

A batch of dry mix consisting of 0.50 kg metronidazole, 1.00 kg microcrystalline cellulose ("MCC"), 0.50 kg lactose and 0.50 kg croscarmellose sodium (Ac-Di-Sol^{™}) was prepared. The optimal amount of water for the dry mix was determined to be 5.10 kg. 90 % (4.59 kg) of the optimal amount of water was added to the dry mix and a portion of the resultant mixture processed as in Example 1. After processing, a small sample of the resultant pellets was retained and the remaining pellets returned to the remaining portion of the mixture. A further 5 % (0.26 kg) of the optimal amount of water was mixed with the mixture and a portion of the new mixture processed as in Example 1. This procedure was repeated a further three times so that results of pellet production runs were obtained for mixtures having 90 wt %, 95 wt %, 100 wt %, 105 wt % or 110 wt % of the optimal amount of water. The results of the five pellet production runs are indicated in Table 1.

**TABLE 1**

| Formulation | | Results | | | |
|---|---|---|---|---|---|
| **Material** | **Amount (kg)** | **Mixture** | **Extrudate** | **Pellets** | **Processing** |
| MCC | 1.00 | a) 90% water added. | Looked normal. | Smaller than normal. Retained sample. | Normal. |
| Ac-Di-Sol* | 0.50 | b) Added further 5% water. | Looked normal. | Smaller than normal. Visible difference. Retained sample. | Normal. |
| Metronidazole | 0.50 | c) Added further 5% water. | Normal. | Good, slightly larger than previous run. Retained sample. | Normal. |
| Lactose | 0.50 | d) Added further 5% water. Wet mix binding. Sticking in lumps. | Longer than normal strands. | Larger than normal pellets. Retained sample. | Slightly more sticky. |
| Water | 5.10 (100%) | e) Added further 5% water. More lumpy, more sticky. | Very long strands. | Very large and uneven. | Sticks to equipment. |

| | | | | | |
|---|---|---|---|---|---|
| * Crosscarmellose Sodium | | | | | |

The results indicate not only that pellets comprising an active component other than prednisolone sodium metasulphobenzoate may be made and but also that the size of metronidazole pellets may be controlled by controlling the amount of water present. In this connection, the results further indicate that each increase in the amount of water, increases the average size of the pellets produced.

### Example 7 - 40 wt% metronidazole (no lactose)

A batch of dry mix consisting of 1.00 kg metronidazole, 1.00 kg MCC and 0.50 kg croscarmellose sodium (Ac-Di-Sol^{™}) was prepared. The dry mix of Example 7 was similar to that of Example 6 except that the lactose in Example 6 was replaced with further metronidazole. The optimal amount of water for the dry mix was again determined to be 5.10 kg. -84 % (4.3 kg) of the optimal amount of water was added to the dry mix and a portion of the resultant mixture processed as in Example 1. After processing, a small sample of the resultant pellets was retained and the remaining pellets returned to the remaining portion of the mixture. A further -10 % (0.5 kg) of the optimal amount of water was mixed with the mixture and a portion of the new mixture processed as in Example 1. After processing, a small sample of the resultant pellets was retained and the remaining pellets returned to the remaining portion of the mixture. A further ~6 % (0.3 kg) of the optimal amount of water (total 100 %) was mixed with the mixture and a portion of the further new mixture processed as in Example 1. The results of the three pellet production runs are indicated in Table 2.

**TABLE 2**

| Formulation | | Results | | | |
|---|---|---|---|---|---|
| **Material** | **Amount (kg)** | **Mixture** | **Extrudate** | **Pellets** | **Processing** |
| MCC | 1.00 | a) 4.3kg water added. | Looked normal. | Smaller than normal. Retained sample. | Normal. |
| Ac-Di-Sol* | 0.50 | b) Added further 0.5kg water. | Looked normal. | Slightly smaller than normal. Retained sample. | Normal. |
| Metronidazole | 1.00 | c) Added further 0.3kg water. | Normal. | Good, normal size. | Normal. Dried batch retained. |
| Water | 5.10 | | | | |

| | | | | | |
|---|---|---|---|---|---|
| * Crosscarmellose Sodium | | | | | |

The results indicate not only that pellets comprising an active component other than prednisolone sodium metasulphobenzoate may be made and but also that the size of the metronidazole pellets may be controlled by controlling the amount of water present. As in Example 6, the results further indicate that each increase in the amount of water, increases the average size of the pellets produced.

### Example 8 - 20 wt % paracetamol

A batch of dry mix consisting of 1.00 kg paracetamol, 2.00 kg MCC, 1.00 kg lactose and 1.00 kg croscarmellose sodium (Ac-Di-Sol^{™}) was prepared. The optimal amount of water for the dry mix was determined to be 9.50 kg. 100 % (9.5 kg) of the optimal amount of water was added to the dry mix and a portion of the resultant mixture processed as in Example 1. After processing, a small sample of the resultant pellets was retained and the remaining pellets returned to the remaining portion of the mixture. A further 5 % (~0.48 kg) of the optimal amount of water was mixed with the mixture and a portion of the new mixture processed as in Example 1. After processing, a small sample of the resultant pellets was retained and the remaining pellets returned to the remaining portion of the mixture. A further 5 % (∼0.48 kg) of the optimal amount of water (total -10.5 kg) was mixed with the mixture and a portion of the further new mixture processed as in Example 1. The results of the three pellet production runs are indicated in Table 3.

**TABLE 3**

| Formulation | | Results | | | |
|---|---|---|---|---|---|
| **Material** | **Amount (kg)** | **Mixture** | **Extrudate** | **Pellets** | **Processing** |
| MCC | 2.00 | a) 100% water added. | Looked normal. | Normal size range. Retained sample. | Normal. |
| Ac-Di-Sol* | 1.00 | b) Added further 5% water. | Looked normal. | Slightly larger than normal. Retained sample. | Normal. |
| Paracetamol | 1.00 | c) Added further 5% water. | Normal. | Larger pellets. Retained sample. | Normal. |
| Lactose | 1.00 | | | | |
| Water | 9.50 (100%) | | | | |

| | | | | | |
|---|---|---|---|---|---|
| * Crosscarmellose Sodium | | | | | |

The results indicate not only that pellets comprising an active component other than prednisolone sodium metasulphobenzoate or metronidazole may be made and but also that the size of the paracetamol pellets may be controlled by controlling the amount of water present. As in Examples 6 and 7, the results further indicate that each increase in the amount of water, increases the average size of the pellets produced.

## Claims

1. A process for the production of particles for use in a pharmaceutical composition, said process comprising:
mixing water with a component composition comprising at least a rheology modifying agent and a therapeutically active agent, to produce a paste, wherein the rheology modifying agent is present in an amount of at least 10 wt % of the component composition and the therapeutically active agent is present in an amount of more than 0 wt % to 90 wt % of the component composition;
extruding at least a portion of the paste to form extrudate;
spheronising at least a portion of the extrudate to form spheronised particles; and
drying at least a portion of the spheronised particles, **characterised in that** the process comprises:
controlling the amount of water used with the component composition to be from 180 wt % to 190 wt % of the component composition, so as to obtain a particle size distribution such that 80% to 98% of the particles have a diameter from 800 to 1500 µm.

2. A process as claimed in Claim 1 wherein the amount of water used with the component composition is controlled so as to obtain a particle size distribution such that 90% to 98% of particles have a diameter between the range of 800 to 1500 µm.

3. A process as claimed in Claim 1 or Claim 2 wherein the amount of water used with the component composition is controlled so as to obtain a particle size distribution such that 95% to 98% of particles have a diameter between the range of 800 to 1500 µm.

4. A process as claimed in any of the preceding claims wherein the composition further comprises a therapeutically active compound selected from peptides, polypeptides, proteins, interferons, TNF antagonists, protein and peptide agonists and antagonists of the immune system, hormones, cytokines and cytokine agonists and antagonists, analgesics, antipyretics, antibacterial and antiprotozoal agents, anti-infective agents, antibiotics, antiviral agents, antifungal agents, antimalarial agents, anti-inflammatory agents, steroids, probiotics and prebiotics, opiate agonists and antagonists, bisphosphonates, anticancer and cytotoxic agents, immunomodulators, antiparasitic agents and pharmacologically acceptable salts and derivatives of each of these actives.

5. A process as claimed in any of the preceding claims wherein the composition further comprises a therapeutically active compound selected from erythropoietin, human growth hormone, metronidazole, albenazole, mebendazole, prazinquantel, clarithromycin, gentamycin, ciprofloxacin, rifabutin, 5-aminosalicylic acid, 4-aminosalicylic acid, balsalazide, prednisolone metasulphobenzoate, α-amylase, paracetamol, metformin, cyclophosphamide, cisplatin, vincristine, methotrexate, azathioprine and cyclosporin or pharmacologically acceptable salts or derivatives thereof.

6. A process as claimed in Claim 4 or Claim 5 wherein the therapeutically active compound is prednisolone or a pharmacologically acceptable salt or derivative thereof.

7. A process as claimed in Claim 4 or Claim 5 wherein the therapeutically active compound is metronidazole or a pharmacologically acceptable salt or derivative thereof.

8. A process as claimed in Claim 4 or Claim 5 wherein the therapeutically active compound is erythropoetin or a pharmacologically acceptable salt or derivative thereof.

9. A process as claimed in any of the preceding claims wherein the rheology modifying agent is selected from croscarmellose sodium; starch; hydroxypropyl methylcellulose; crospovidone; or sodium starch glycolate.

10. A process as claimed in any of the preceding claims wherein the rheology modifying agent is present in an amount of between from 10 to 40 wt % of the component composition.

11. A process as claimed in any of the preceding claims wherein the component composition further comprises sugar.

12. A process as claimed in Claim 11 wherein the sugar is lactose monohydrate.

13. A process as claimed in Claim 11 or Claim 12 wherein the sugar is present in an amount of between from 30 to 50 wt % of the component composition.

14. A process as claimed in any of the preceding claims wherein the component composition further comprises cellulose.

15. A process as claimed in Claim 14 wherein the cellulose is microcrystalline cellulose.

16. A process as claimed in Claim 14 or Claim 15 wherein the cellulose is present in an amount of between from 35 to 45 wt % of the component composition.

17. A process as claimed in any of Claims 1 to 3 wherein the composition consists essentially of prednisolone or a pharmacologically acceptable salt or derivative thereof, a rheology modifying agent, sugar and cellulose.

18. A process as claimed in any of Claims 1 to 3 wherein the composition consists essentially of metronidazole or a pharmacologically acceptable salt or derivative thereof, a rheology modifying agent, sugar and cellulose.

19. A process as claimed in any of Claims 1 to 3 wherein the composition consists essentially of erythropoetin or a pharmacologically acceptable salt or derivative thereof, a rheology modifying agent, sugar and cellulose.

20. A process as claimed in any of the preceding claims wherein said at least a portion of the extrudate is spheronised using a spheroniser having a spheronising plate, said process comprising controlling the speed of rotation of the spheronising plate to from 125 rpm to 1800 rpm.

## Patentansprüche

1. Verfahren zur Herstellung von Teilchen zur Verwendung in einer pharmazeutischen Zusammensetzung, wobei das besagte Verfahren Folgendes umfasst:
Das Mischen von Wasser mit einer Komponentenzusammensetzung zur Herstellung einer Paste, die mindestens ein rheologieveränderndes Mittel und einen therapeutischen Wirkstoff enthält, wobei das rheologieverändernde Mittel in einem Anteil von mindestens 10 Gew.-% der Komponentenzusammensetzung und der therapeutische Wirkstoff in einem Anteil von mehr als 0 Gew.-% bis 90 Gew.-% der Komponentenzusammensetzung vorliegt;
Das Extrudieren von mindestens einem Teil der Paste zur Bildung des Extrudats;
Die Sphäronisierung (Granulatabrundung) von mindestens einem Teil des Extrudats zur Bildung von sphäronisierten Teilchen; und
Die Trocknung zumindest eines Teils der sphäronisierten Teilchen, **gekennzeichnet durch** die in dem Verfahren umfassten Komponenten:
Die Steuerung des bei der Komponentenzusammensetzung verwendeten Anteils an Wasser, der von 180 Gew.-% bis 190 Gew.-% der Komponentenzusammensetzung reichen darf, damit eine Teilchengrößenverteilung erreicht wird, bei der 80% bis 98% der Teilchen einen Durchmesser von 800 bis 1500 µm aufweisen.

2. Verfahren nach Anspruch 1, bei dem der Anteil des bei der Komponentenzusammensetzung verwendeten Wassers gesteuert wird, um eine Teilchengrößenverteilung zu erhalten, bei der 90% bis 98% der Teilchen einen Durchmesser im Bereich zwischen 800 bis 1500 µm aufweisen.

3. Verfahren nach Anspruch 1 oder 2, bei dem der Anteil des bei der Komponentenzusammensetzung verwendeten Wassers gesteuert wird, um eine Teilchengrößenverteilung zu erhalten, bei der 95% bis 98% der Teilchen einen Durchmesser im Bereich zwischen 800 bis 1500 µm aufweisen.

4. Verfahren nach einem der vorhergehenden Ansprüche, bei dem die Zusammensetzung ferner einen therapeutischen Wirkstoff enthält, ausgewählt aus Peptiden, Polypeptiden, Proteinen, Interferonen, TNF-Antagonisten, Protein- und Peptid-Agonisten und - Antagonisten des Immunsystems, Hormonen, Zytokinen und Zytokin-Agonisten und - Antagonisten, Analgetika, Antipyretika, antibakteriellen Mitteln und Antiprotozoika, Antiinfektiva, Antibiotika, antiviralen Mitteln, Antimykotika, Antimalariamitteln, entzündungshemmenden Mitteln, Steroiden, Probiotika, Präbiotika, Opiat-Agonisten und - Antagonisten, Bisphosphonaten, Antikrebs- und zytotoxischen Mitteln, Immunmodulatoren, Antiparasitika und pharmakologisch verträglichen Salzen und Derivaten von jedem dieser Wirkstoffe.

5. Verfahren nach einem der vorhergehenden Ansprüche, bei dem die Zusammensetzung ferner einen therapeutischen Wirkstoff enthält, der ausgewählt wurde aus Erythropoetin, menschlichen Wachstumshormonen, Metronidazol, Albendazol, Mebendazol, Praziquantel, Clarithromycin, Gentamycin, Ciprofloxacin, Rifabutin, 5-Aminosalicylsäure, 4-Aminosalicylsäure, Balsalazid, Prednisolonmetasulfobenzoat, α-Amylase, Paracetamol, Metformin, Cyclophosphamid, Cisplatin, Vincristin, Methotrexat, Azathioprin und Cyclosporin oder pharmakologisch verträglichen Salzen oder Derivaten von diesen Wirkstoffen.

6. Verfahren nach Anspruch 4 oder Anspruch 5, bei dem der therapeutische Wirkstoff Prednisolon oder ein pharmakologisch verträgliches Salz oder Derivat von diesem ist.

7. Verfahren nach Anspruch 4 oder Anspruch 5, bei dem der therapeutische Wirkstoff Metronidazol oder ein pharmakologisch verträgliches Salz oder Derivat von diesem ist.

8. Verfahren nach Anspruch 4 oder Anspruch 5, bei dem der therapeutische Wirkstoff Erythropoetin oder ein pharmakologisch verträgliches Salz oder Derivat von diesem ist.

9. Verfahren nach einem der vorhergehenden Ansprüche, bei dem das rheologieverändernde Mittel ausgewählt wurde aus Croscarmellose-Natrium, Stärke; Hydroxypropylmethylcellulose, Crospovidon oder Natriumstärkeglykolat.

10. Verfahren nach einem der vorhergehenden Ansprüche, bei dem das rheologieverändernde Mittel in einem Anteil von zwischen 10 bis 40 Gew.-% der Komponentenzusammensetzung vorliegt.

11. Verfahren nach einem der vorhergehenden Ansprüche, bei dem die Komponentenzusammensetzung ferner Zucker umfasst.

12. Verfahren nach Anspruch 11, bei dem es sich beim Zucker um Lactosemonohydrat handelt.

13. Verfahren nach Anspruch 11 oder Anspruch 12, bei dem der Zucker in einem Anteil von zwischen 30 bis 50 Gew.-% der Komponentenzusammensetzung vorliegt.

14. Verfahren nach einem der vorhergehenden Ansprüche, bei dem die Komponentenzusammensetzung ferner Cellulose umfasst.

15. Verfahren nach Anspruch 14, bei dem die Cellulose mikrokristalline Cellulose ist.

16. Verfahren nach Anspruch 14 oder Anspruch 15, bei dem die Cellulose in einem Anteil von zwischen 35 bis 45 Gew.-% der Komponentenzusammensetzung vorliegt.

17. Verfahren nach einem der Ansprüche 1 bis 3, bei dem die Zusammensetzung im Wesentlichen aus Prednisolon oder einem pharmakologisch verträglichen Salz oder Derivat von diesem, einem rheologieverändernden Mittel, Zucker und Cellulose besteht.

18. Verfahren nach einem der Ansprüche 1 bis 3, bei dem die Zusammensetzung im Wesentlichen aus Metronidazol oder einem pharmakologisch verträglichen Salz oder Derivat von diesem, einem rheologieverändernden Mittel, Zucker und Cellulose besteht.

19. Verfahren nach einem der Ansprüche 1 bis 3, bei dem die Zusammensetzung im Wesentlichen aus Erythropoetin oder einem pharmakologisch verträglichen Salz oder Derivat von diesem, einem rheologieverändernden Mittel, Zucker und Cellulose besteht.

20. Verfahren nach einem der vorhergehenden Ansprüche, bei dem zumindest ein Teil des Extrudats mit einem Sphäronisator mit einer Sphäronisierungsscheibe sphäronisiert wird und bei dem die Rotationsgeschwindigkeit der Sphäronisierungsscheibe im Bereich ab 125 UpM bis auf 1800 UpM gesteuert wird.

## Revendications

1. Procédé de production de particules destinées à être utilisées dans une composition pharmaceutique, ledit procédé comprenant les étapes consistant à :
mélanger de l'eau avec une composition de composants contenant au moins un agent modificateur de rhéologie et un principe thérapeutiquement actif, pour produire une pâte, ledit agent modificateur de rhéologie étant présent en une quantité représentant au moins 10 % en poids de la composition de composants et le principe thérapeutiquement actif étant présent en une quantité représentant plus de 0 % en poids à 90 % en poids de la composition de composants ;
extruder au moins une partie de la pâte pour produire un extrudat ;
sphéroniser au moins une partie de l'extrudat pour produire des particules sphéronisées ; et
sécher au moins une partie des particules sphéronisées, **caractérisé en ce que** le procédé comprend :
le contrôle de la quantité d'eau utilisée avec la composition de composants afin qu'elle représente de 180 % en poids à 190 % en poids de la composition de composants de façon à obtenir une distribution granulométrique telle que 80 % à 98 % des particules ont un diamètre de 800 à 1500 µm.

2. Procédé selon la revendication 1, ladite quantité d'eau utilisée avec la composition de composants étant contrôlée de façon à obtenir une distribution granulométrique telle que 90 % à 98 % des particules ont un diamètre compris dans la plage de 800 à 1500 µm.

3. Procédé selon la revendication 1 ou la revendication 2, ladite quantité d'eau utilisée avec la composition de composants étant contrôlée de façon à obtenir une distribution granulométrique telle que 95 % à 98 % des particules ont un diamètre compris dans la plage de 800 à 1500 µm.

4. Procédé selon l'une quelconque des revendications précédentes, ladite composition contenant en outre un principe thérapeutiquement actif choisi parmi les peptides, les polypeptides, les protéines, les interférons, les antagonistes du TNF, les agonistes et antagonistes peptidiques et protéiques du système immunitaire, les hormones, les cytokines et les agonistes et antagonistes de cytokines, les analgésiques, les antipyrétiques, les agents antibactériens et antiprotozoaires, les agents anti-infectieux, les antibiotiques, les agents antiviraux, les agents antifongiques, les agents antipaludiques, les agents anti-inflammatoires, les stéroïdes, les probiotiques et les prébiotiques, les agonistes et antagonistes d'opiacés, les biphosphonates, les agents anticancéreux et cytotoxiques, les immunomodulateurs, les agents antiparasitaires et les sels et dérivés pharmacologiquement acceptables de chacun de ces principes actifs.

5. Procédé selon l'une quelconque des revendications précédentes, ladite composition contenant en outre un principe thérapeutiquement actif choisi parmi l'érythropoïétine, l'hormone de croissance humaine, le métronidazole, l'albénazole, le mébendazole, le prazinquantel, la clarithromycine, la gentamycine, la ciprofloxacine, la rifabutine, l'acide 5-aminosalicylique, l'acide 4-aminosalicylique, le basalazide, le prédnisolone métasulphobenzoate, l'α-amylase, le paracétamol, la metformine, le cyclophosphamide, la cisplatine, la vincristine, le méthotrexate, l'azathioprine et la cyclosporine ou leurs sels ou dérivés pharmacologiquement acceptables.

6. Procédé selon la revendication 4 ou la revendication 5, ledit principe thérapeutiquement actif étant la prédnisolone ou un sel ou dérivé pharmacologiquement acceptable de celle-ci.

7. Procédé selon la revendication 4 ou la revendication 5, ledit principe thérapeutiquement actif étant le métronidazole ou un sel ou dérivé pharmacologiquement acceptable de celui-ci.

8. Procédé selon la revendication 4 ou la revendication 5, ledit principe thérapeutiquement actif étant l'érythropoïétine ou un sel ou dérivé pharmacologiquement acceptable de celui-ci.

9. Procédé selon l'une quelconque des revendications précédentes, ledit agent modificateur de rhéologie étant choisi parmi le croscarmellose de sodium, l'amidon, l'hydroxypropylméthylcellulose, la crospovidone ou le glycolate d'amidon sodique.

10. Procédé selon l'une quelconque des revendications précédentes, ledit agent modificateur de rhéologie étant présent en une quantité représentant de 10 à 40 % en poids de la composition de composants.

11. Procédé selon l'une quelconque des revendications précédentes, ladite composition de composants contenant en outre du sucre.

12. Procédé selon la revendication 11, ledit sucre étant le lactose monohydraté.

13. Procédé selon la revendication 11 ou la revendication 12, ledit sucre étant présent en une quantité représentant de 30 à 50 % en poids de la composition de composants.

14. Procédé selon l'une quelconque des revendications précédentes, ladite composition de composants contenant en outre de la cellulose.

15. Procédé selon la revendication 14, ladite cellulose étant de la cellulose monocristalline.

16. Procédé selon la revendication 14 ou la revendication 15, ladite cellulose étant présente en une quantité représentant de 35 à 45 % en poids de la composition de composants.

17. Procédé selon l'une quelconque des revendications 1 à 3, ladite composition se composant essentiellement de prédnisolone ou d'un sel ou dérivé pharmacologiquement acceptable de celle-ci, d'un agent modificateur de rhéologie, de sucre et de cellulose.

18. Procédé selon l'une quelconque des revendications 1 à 3, ladite composition se composant essentiellement de métronidazole ou d'un sel ou dérivé pharmacologiquement acceptable de celui-ci, d'un agent modificateur de rhéologie, de sucre et de cellulose.

19. Procédé selon l'une quelconque des revendications 1 à 3, ladite composition se composant essentiellement d'érythropoïétine ou d'un sel ou dérivé pharmacologiquement acceptable de celle-ci, d'un agent modificateur de rhéologie, de sucre et de cellulose.

20. Procédé selon l'une quelconque des revendications précédentes, ladite au moins partie de l'extrudat étant sphéronisée au moyen d'un sphéroniseur possédant un plateau de sphéronisation, ledit procédé comprenant le contrôle de la vitesse de rotation du plateau de sphéronisation de 125 rpm à 1800 rpm.
